# EUROPEAN PATENT APPLICATION

(11) **EP 4 811 367 A1**
(43) Date of publication of application: **23.09.2026**
(21) Application number: 25164322.7
(22) Date of filing: 18.03.2025
(51) Int. Cl.: G16H 10/40, G16H 20/30, G16H 20/70, A61B 5/00, G16H 50/20, A61N 1/36

(54) **COMPUTER-IMPLEMENTED METHOD, RESPIRATORY SUPPORT DEVICE, AND COMPUTER PROGRAM PRODUCT**

(71) Applicant: Koninklijke Philips N.V., 5656 AE Eindhoven (NL)
(72) Inventor: KUHN, Jasmin, Eindhoven (NL); FERREIRA DOS SANTOS DA FONSECA, Pedro Miguel, Eindhoven (NL); OVEREEM, Sebastiaan, 5656AG Eindhoven (NL); VAN GILST, Merel Marietje, Eindhoven (NL)
(74) Representative: Philips Intellectual Property & Standards

(57) **Abstract**

There is provided a computer-implemented method comprising receiving subjective sleep time data representative of a subjective sleep time of a subject over multiple days; receiving objective sleep time data representative of an objective sleep time of the subject over the multiple days; determining a sleep time misperception based on the subjective sleep time data and the objective sleep time data; performing a classification of the subject to one of multiple sleep perception classes based on the sleep time misperception; and generating an output signal representative of a suggested action based on the classification. The suggested action relates to providing sleep-related therapy to the subject.

## Description

### FIELD OF THE INVENTION

The invention relates to a computer-implemented method relating to determining sleep misperception. Further, the invention relates to a respiratory support device for providing a pressurized airflow to a subject. Further, the invention relates to a further computer-implemented method relating to suggesting an action for subjects in a priority class.

### BACKGROUND OF THE INVENTION

Sleep-disordered breathing (SDB) conditions, such as obstructive sleep apnea, OSA, and central sleep apnea, CSA, are becoming increasingly common, and are particularly prevalent in older people, people with a high body mass index, smokers, heavy drinkers and people with conditions such as coronary artery disease, hypertension and diabetes mellitus.

SDB conditions are often treated using positive airway pressure (PAP) therapy, in which pressurized air is provided to a subject to keep the subject's airways open. The pressurized air is provided to the subject via a patient interface, such as a nasal pillow, an oronasal mask, or a full-face mask.

Despite the health benefits of PAP therapy for subjects suffering from SDB, it is difficult for many subjects to adhere to PAP therapy. The feeling of the patient interface and breathing through the pressurized air causes discomfort to the subject. This discomfort makes it more difficult for subjects to fall asleep and to stay asleep.

The difficulty of adhering to PAP therapy depends on how subjects perceive the amount of sleep they have. Subjects that underestimate their amount of sleep, i.e., they think they sleep less than they do, often have a reduced quality of life, and may have an increased anxiety about the perceived lack of sleep. As a result, these subjects may consider the PAP therapy to be ineffective and become less adherent, or may completely stop PAP therapy.

To diagnose SDB or to perform a titration session to set up PAP therapy, it is common that the subject spends a night at a hospital or sleep center, while connected to a polysomnograph (PSG). The PSG provides a large amount of information about sleep parameters of the subject. However, no accurate information about sleep misperception is obtained. For professional caretakers, it is difficult to determine which subjects suffer from sleep underestimation.

### SUMMARY OF THE INVENTION

It is an objective of the invention to provide improved sleep disordered breathing (SDB) therapy.

According to a first aspect, there is provided a computer-implemented method comprising receiving subjective sleep time data representative of a subjective sleep time of a subject over multiple days. The computer-implemented method comprises receiving objective sleep time data representative of an objective sleep time of the subject over the multiple days. The computer-implemented method comprises determining a sleep time misperception based on the subjective sleep time data and the objective sleep time data. The computer-implemented method comprises performing a classification of the subject to one of multiple sleep perception classes based on the sleep time misperception. The computer-implemented method comprises generating an output signal representative of a suggested action based on the classification. The suggested action relates to providing sleep-related therapy to the subject.

By receiving the subjective sleep time data and the objective sleep time data over multiple days, any sleep time misperception can be determined based with an improved accuracy. Based on the accurate sleep time misperception, the subject is classified into one of the sleep perception classes. Based on the classification of the subject, the computer-implemented method generates the output signal to provide the suggested action about providing the sleep-related therapy. In case the subject has no sleep underestimation, the subject is able to start or to continue with the SDB therapy, such as PAP therapy. SDB therapy is a form of sleep-related therapy. A subject without sleep underestimation has a good chance of adhering to the SDB therapy. In case the subject has sleep underestimation, the chance of successfully starting or continuing with the SDB therapy is low. Instead, the subject should start with a sleep-related therapy that improves the perceived quality of sleep. Such sleep-related therapy comprises, for example, behavioral therapy or the use of sleep medication. By improving the quality of sleep via the sleep-related therapy, the subject has a higher chance of adhering to the SDB therapy. Based on the output signal, the clinician is informed about the suggested action based on the classification. This directs the clinician to prescribe the suggested action. The clinician is able to directly prescribe SDB therapy to subjects without sleep underestimation. The clinician is able to prescribe sleep-related therapy to improve sleep, either prior to or in combination with SDB therapy, to subjects with sleep underestimation. As a result, adherence to the SDB therapy is improved, so an improved SDB therapy is obtained.

The subjective sleep time data is representative of the subjective sleep time of the subject over multiple days. Daily subjective sleep time is, for example, assessed through the use of an application such as a questionnaire or a sleep diary. In such an application, the subject notes down his/her subjective sleep duration for the previous night each morning upon waking up. For example, the notes are automatically interpreted e.g., by the means of optical character recognition or other image analysis tools. For example, the subject inputs the subjective sleep time by means of an interface, such as a touch screen display, or a mobile device, or a therapy device such as a PAP therapy device. For example, the subject provides the subjective sleep time as a number of hours and a number of minutes, such as 6 hours and 42 minutes. For example, the subject provides the subjective sleep time as a number of hours and a number of quarters of an hour, such as 6 hours and 15 minutes, or 7 hours and 30 minutes. For example, the subject selects the subjective sleep time from a plurality of ranges. The ranges are, for example, less than 5 hours, 5-6 hours, 6-7 hours, 7-8 hours, and more than 8 hours.

The objective sleep time data is representative of the objective sleep time of the subject over the multiple days. The objective sleep time comprises, for example, the time the subject is asleep during multiple nights. The objective sleep time, for example, excludes time that sleep of the subject is interrupted during the night. The subject may be aware that the sleep is interrupted or may be unaware that the sleep is interrupted. The objective sleep time data is, for example, based on measurement data obtained from the subject. For example, the measurement data comprises cardiac data representative of cardiac activity. Methods are known to determine whether a subject is asleep or awake based on cardiac activity. For example, the cardiac data is generated by a wearable device, such as a photoplethysmographic (PPG) sensor, an electrocardiographic (ECG) sensor, or a seismocardiographic sensor. For example, the seismocardiographic sensor comprises an accelerometer adapted to be mounted on the subject and adapted to detect accelerations of the subject caused by heart beats. For example, the cardiac data is generated by a remote PPG sensor, such as an infrared camera. For example, the cardiac data is generated by a bed-related sensor based on ballistocardiography or seismocardiography.

For example, the measurement data comprises respiratory data representative of respiratory activity. Methods are known to determine whether a subject is asleep or awake based on respiratory data. For example, the respiratory data is generated by a wearable device, such as a photoplethysmographic (PPG) sensor, or a seismocardiographic sensor. For example, the seismocardiographic sensor comprises an accelerometer adapted to be mounted on the subject and adapted to detect accelerations of the subject caused by breathing. For example, the respiratory data is generated by a remote RF sensor, such as a Doppler Radar. For example, the respiratory data is generated by a bed-related sensor based on ballistocardiography or seismocardiography.

For example, the measurement data comprises neurological data representative of neural activity of the subject. For example, the neurological data is based on electroencephalography (EEG), electrooculography (EOG), electromyography (EMG), or any combination thereof.

For example, the objective sleep time data is based on sleep stage information of the subject. The sleep stage information provides information about the sleep stage the subject is in during a night's sleep. The sleep stages include, for example, light sleep (N1, N2), deep sleep (N3), REM-sleep, and Wake. By adding the periods the subject is in a sleep stage other than Wake, the objective sleep time is determined. For example, the sleep stage information is determined using the method as disclosed in WO2024200134A1, hereby incorporated by reference. For example, the sleep stage information is determined using the method as disclosed in US patent US10524674B2, hereby incorporated by reference.

The sleep time misperception is based on the subjective sleep time data and the objective sleep time data. For example, the sleep time misperception is based on a difference between the objective sleep time data and the subjective sleep time data. A positive sleep time misperception is representative of an underestimation of the subjective sleep time. A negative sleep time misperception is representative of an overestimation of the subjective sleep time. The more the sleep time misperception deviates from zero, the larger the sleep time misperception is. For example, the degree of sleep misperception is expressed as the sleep misperception index. The sleep misperception index MI is calculated as follows: $MI = \left(OST-SST\right) / OST$ wherein OST is the objective sleep time, and wherein SST is the subjective sleep time. The sleep misperception index MI is further described in "Measuring the error in sleep estimation in normal subjects and in patients with insomnia", Manconi et al., J. Sleep Res. 2010, 19, 478-486. For example, the sleep time misperception is expressed as a ratio between the objective sleep time and the subjective sleep time.

The subject is classified as one of multiple sleep perception classes based on the sleep time misperception. In an embodiment, there are only two sleep perception classes. For example, the two sleep perception classes are one class representing sleep misperception, and one class representing no sleep misperception. In case the subject is classified as having no sleep misperception, the output signal suggests the clinician to start or continue SDB therapy aimed at optimal treatment of the SDB. As the subject has no sleep misperception, the subject has a high chance of adhering to the SDB therapy. In case the subject is classified as having sleep misperception, the output signal suggests taking another action to provide sleep-related therapy. For example, the suggested action is to reduce the intensity of the SDB therapy to improve adherence, at the expense of sub-optimal treatment of the SDB. For example, the suggested action is to perform further examination of the subject to obtain more information about the sleep misperception. For example, the two sleep perception classes are one class representing sleep underestimation, and one class representing no sleep underestimation. In case the subject is classified as having no sleep underestimation, the output signal suggests the clinician to start or continue SDB therapy aimed for optimal treatment of the SDB. As the subject has no sleep underestimation, the subject has a high chance of adhering to the SDB therapy. In case the subject is classified as having sleep underestimation, the output signal suggests taking another action to provide sleep-related therapy. For example, the suggested action is to reduce the intensity of the SDB therapy to improve adherence, at the expense of sub-optimal treatment of the SDB, or to improve the sleep underestimation by improving sleep quality.

For example, the output signal causes the suggested action to be displayed on a computer monitor, or a mobile device, or on any other type of display.

In an embodiment, the sleep-related therapy comprises sleep disordered breathing therapy, or insomnia therapy, or a combination of sleep disordered breathing therapy and insomnia therapy.

According to the embodiment, the output signal suggests an action relating to sleep disordered breathing therapy and/or insomnia therapy. The sleep disordered breathing therapy comprises, for example, providing a pressurized airflow to the airway of the subject with a respiratory support device. The action relates, for example, to adjusting a pressure or a pressure profile of the pressurized airflow. For example, the action relates to reducing the pressure or reducing a ramp-up of pressure to increase comfort to the subject. For example, the respiratory support device is a positive airway pressure (PAP) device, such as a CPAP device, a BiPAP device, or an autoPAP device. The sleep disordered breathing therapy comprises, for example, providing positional therapy with a positional therapy device. The positional therapy device is adapted to detect whether the subject is sleeping in a supine position. In the supine position, the SDB is worse than when the subject is sleeping in a non-supine position, such as on the side or prone. In case the positional therapy device detects the subject is sleeping in the supine position, the positional therapy device causes an alarm to be triggered. The alarm induces the subject to change to a different position than the supine position. The sleep disordered breathing therapy comprises, for example, advancing the mandible of the subject with a mandibular advancement device (MAD-device). By advancing the mandible relative to the upper jaw, obstructions in the airway during sleep are reduced.

The insomnia therapy is a therapy that helps the subject to sleep more, for example, by helping the subject to fall asleep faster and/or by helping the subject to remain asleep longer. The insomnia therapy is beneficial, for example, for a subject diagnosed with insomnia, or for a subject having some insomnia symptoms. For example, the insomnia therapy comprises cognitive behavioral therapy for insomnia (CBT-I). CBT-I is a therapy technique for treating insomnia. CBT-I aims to improve sleep habits and sleep behaviors by changing thoughts and behaviors of the subject that prevents the subject from sleeping well. For example, the insomnia therapy comprises the use of sleep medication to help the subject to fall asleep more easily, or to remain asleep longer. For example, the insomnia therapy comprises the use of anxiety medication to help the subject to have less anxiety. When the subject has less anxiety, the subject is better able to fall asleep. For example, the insomnia therapy comprises pain treatment. A subject having pain may have difficulties sleeping. By reducing the pain with pain treatment, the subject is better able to sleep. The pain treatment comprises, for example, the use of pain medication or physical exercises.

In an embodiment, the multiple sleep perception classes comprise at least one overestimation class and at least one underestimation class. The overestimation class represents subjects indicating more subjective sleep time than the objective sleep time. The underestimation class represents subjects indicating less subjective sleep time than the objective sleep time.

According to the embodiment, the subject may be classified in the overestimation class or in the underestimation class. The inventors have discovered that subjects in these two classes have different characteristics. A subject suffering from SDB and that has sleep overestimation likely has a high AHI and a disrupted sleep architecture. A high AHI means that the subject has many apnea events or hypopnea events per unit of time. Because of the disrupted sleep architecture, the subject may suffer from daytime sleepiness. However, the subject thinks he/she sleeps more than he/she does, as is indicated by the sleep overestimation. As a result, the subject is less likely to be anxious that undergoing SDB therapy would cause insufficient sleep. The suggested action therefore is to optimize the SDB therapy to maximally reduce the high AHI. On the other hand, a subject suffering from SDB and that has sleep underestimation likely experiences insomnia symptoms. The subject would likely experience SDB therapy, especially intensive SDB therapy, as something that makes it even more difficult to sleep sufficiently. The suggested action therefore is to improve the sleep underestimation, for example, by improving insomnia. The suggested action is, for example, aimed at only improving the insomnia. The suggested action is, for example, primarily aimed at improving the insomnia, and secondary aimed at improving the SDB.

In an embodiment, in case the classification is to the overestimation class, the suggested action relates to increasing an intensity of sleep disordered breathing therapy. In case the classification is to the underestimation class, the suggested action relates to providing cognitive behavior therapy for insomnia (CBT-I).

According to the embodiment, a subject classified in the overestimation class likely has a severe form of SDB, but experiences sufficient sleep time. The suggested action relates to providing SDB therapy with an increased intensity. The increased intensity reduces the severity of the SDB, whereas the subject experience sufficient sleep time while undergoing the intense SDB therapy. As a result, the subject is able to adhere to the SDB therapy. A subject classified in the underestimation class would have difficulties with undergoing SDB therapy, because the subject already experiences insufficient sleep time without undergoing SDB therapy. The subject would not adhere to the SDB therapy for a long time. So instead, the suggested action is to provide CBT-I. The CBT-I is aimed to improve the insomnia of the subject. As the CBT-I improves the insomnia, the subject sleeps better and experiences less sleep underestimation. When the sleep underestimation is less than a threshold, the subject may start SDB therapy.

In an embodiment, in case the classification is to the overestimation class, the suggested action relates to increasing an intensity of sleep disordered breathing therapy. In case the classification is to the underestimation class, the suggested action relates to decreasing the intensity of sleep disordered breathing therapy.

In an embodiment, the multiple sleep perception classes comprise at least one severe underestimation class. The severe underestimation class represents subjects indicating less subjective sleep time relative to the objective sleep time than the subjects in the underestimation class. The suggested action relates to providing insomnia therapy and relates to postponing providing sleep disordered breathing therapy.

According to the embodiment, a subject classified in the severe underestimation class estimates the subjective sleep time as being far less than the objective sleep time. Because of the severe underestimation, the subject is unlikely able to adhere to SDB therapy, because the subject would consider the SDB therapy to make it even more difficult to sleep sufficiently. Therefore, the suggested action relates to providing insomnia therapy to improve the underestimation. The suggested action also relates to postponing SDB therapy. In case the subject already undergoes SDB therapy, the suggested action is to stop providing SDB therapy. In case the subject has not yet started with SDB therapy, the suggested action is not to start with SDB therapy. By suggesting starting insomnia therapy, the aim is to improve the insomnia of the subject to such a level that the subject can start using SDB therapy. For example, the level of the insomnia is improved to mild insomnia or light insomnia when the suggested action is to start SDB therapy. For example, the insomnia therapy comprises cognitive behavior therapy for insomnia (CBT-I), or medication.

In an embodiment, in case the classification is to the underestimation class, the action relates to providing both cognitive behavior therapy for insomnia (CBT-I) and sleep disordered breathing therapy.

According to the embodiment, in case the subject is classified in the underestimation class, the subject benefits from CBT-I therapy to improve the insomnia. However, the underestimation is not as bad as, for example, in the severe underestimation class. Therefore, the subject is able to use both the CBT-I therapy and the SDB therapy concurrently. The CBT-I therapy improves the sleep underestimation while the SDB therapy improves the sleep disordered breathing. This improves the health of the subject, while allowing the subject to adhere to the SDB therapy.

In an embodiment, the sleep disordered breathing therapy comprises providing a pressurized airflow to the subject. In case the classification is to the overestimation class, the suggested action relates to providing the pressurized airflow to the subject at a higher pressure than in case the classification is to the underestimation class.

According to the embodiment, the sleep disordered breathing therapy comprises providing a pressurized airflow to the airway of the subject, for example, with a respiratory support device. For example, the respiratory support device is a positive airway pressure (PAP) device, such as a CPAP device, a BiPAP device, or an autoPAP device. The inventors have discovered that many subjects that overestimate the subjective sleep time have a severe form of SDB, such as a high AHI. The suggested action is to provide the pressurized airflow at a higher pressure than for a subject in the underestimation class. The subject in the overestimation class has a health benefit when receiving the higher pressure, which is that the severity of the SDB becomes less. Further, the subject in the overestimation class is less bothered by the increase in pressure compared to a subject in the underestimation class, because the subject in the overestimation class thinks he/she has more sleep than the objective sleep time. So the subject in the overestimation is likely to adhere to the SDB therapy with the higher pressure.

In an embodiment, the multiple sleep perception classes comprise at least one correct estimation class. The correct estimation class represents subjects indicating subjective sleep time matching with the objective sleep time. In case the classification is to the correct estimation class, the suggested action relates to providing sleep disordered breathing therapy and relates to providing no insomnia therapy.

According to this embodiment, there is an overestimation class, an underestimation class, and a correct estimation class. A subject is classified into the correct estimation class in case the subject estimates the subjective sleep time almost the same as the objective sleep time. For example, the subjective sleep time differs from the objective sleep time less than 15% or less than 10% or less than 5%. When the subject has a correct sleep estimation, there is no need to provide insomnia therapy, such as CBT-I, to improve insomnia. Instead, the suggested action is to provide SDB therapy to the subject to treat the SDB. Because the subject has a correct sleep estimation, the subject is likely to adhere to the SDB therapy.

In an embodiment, performing the classification comprises calculating a measure of a tendency and a measure of a variation of the sleep time misperception over the multiple days. The computer-implemented method comprises providing a centroid of each of the multiple sleep perception classes. The computer-implemented method comprises classifying the subject to the sleep perception class having the centroid closest to the measure of the tendency and the measure of the variation.

The inventors have discovered that sleep time misperception determined based on a single-night PSG does not provide accurate information about the sleep misperception of the subject. Many subjects that underestimate sleep time during the single-night PSG overestimate sleep time at home and vice versa. There may be several reasons for this inaccuracy. The subject may sleep differently during the single-night PSG because of the sensors connected to the subject, because of sleeping away from home, or because of anxiety of the whole medical process. Also, the sleep time misperception may vary over nights, so the result of a single night may not be representative over a longer period of time. According to the embodiment, the tendency and the variation of the sleep time misperception are determined over multiple days. By determining over multiple days, the tendency and the variation of the sleep time misperception provide a more accurate prediction of the sleep time misperception of the subject. By classifying the subject to the sleep perception class that has its centroid closest to the measure of tendency and the measure of variation, the subject is classified with increased accuracy. For example, the subject is classified to one of the sleep perception classes by calculating to which centroid the subject is closest within the feature space in terms of Euclidean distance. For example, the measure of tendency is based on the median or the average of the difference between the subjective sleep time and the objective sleep time over the multiple nights. For example, the measure of tendency is based on the median or the average of the sleep misperception index MI. For example, the measure of variation is based on the spread or variation the difference between the subjective sleep time and the objective sleep time over the multiple nights. For example, the measure of variation is based on the standard deviation or the mean absolute deviation (MAD) of the sleep misperception index MI. For example, the measures of tendency and/or variation are based on an absolute difference between the subjective sleep time and the objective sleep time over the multiple nights, or based on other types of sample statistics such as specific percentiles, interquartile ranges, or interpercentile ranges. For example, the multiple nights are at least 2 nights, or at least 4 nights, or at least 7 nights, or at least 10 nights, such as 12 nights, or at least 14 nights.

In a second aspect, there is provided a respiratory support device adapted to provide a pressurized airflow to a subject. The respiratory support device comprises a processor, a sensor input, and a user input. The processor is configured to cause the computer-implemented method according to the first aspect to be carried out. The sensor input is adapted to receive from a sensor a sensor signal representative of a sleep state of the subject. The user input is adapted to receive a user signal representative of the subjective sleep time. The processor is connected to the sensor input and the user input. The processor is configured to determine the objective sleep time data based on the sensor signal. The processor is configured to determine the subjective sleep time data based on the user signal.

According to the second aspect, the respiratory support device is adapted to provide a pressurized airflow to the subject. The pressurized airflow helps to reduce collapse of the subject's airway during sleep. By reducing the collapse of the airway, the SDB is improved. For example, the respiratory support device has a pressure source, such as an impeller or an air pump, to generate the pressurized airflow. The sensor input is generated by a sensor. For example, the sensor is a part of the respiratory support device. For example, the sensor is arranged in a patient interface that couples to the subject to provide the pressurized airflow to the subject. For example, the sensor is a wearable sensor adapted to be worn at the wrist or at the chest or at the neck of the subject. The sensor input is adapted to receive the sensor signal, for example, via a wire or wireless, such as via Bluetooth or Wi-Fi, or any other type of wireless communication. For example, the sensor generates the sensor signal based on measurement data comprising respiratory data, cardiac data, and/or neurological data. For example, the respiratory support device comprises a user interface. The user input is, for example, adapted to receive the user signal from the user interface. In another example, the user provides information about the subjective sleep time via a mobile device. The user input is adapted to communicate with the mobile device, such as via Bluetooth or Wi-Fi, to receive the user signal representative of information about the subjective sleep time.

In an embodiment, the respiratory support device comprises a pressure source adapted to generate the pressurized airflow. The processor is configured to control the pressure source based on the output signal.

According to the embodiment, the pressure source adapts a property of the pressurized airflow based on the output signal. For example, in case the output signal is representative of a suggested action to reduce the intensity of the SDB therapy, the pressure source reduces the pressure of the pressurized airflow. For example, in case the output signal is representative of a suggested action to increase the intensity of the SDB therapy, the pressure source increases the pressure of the pressurized airflow. Based on the output signal, the pressure source is, for example, adapted to change a pressure profile, or a pressure ramp-up, or a pressure ramp-down of the pressurized airflow. For example, the pressure source is adapted to change the pressurized airflow within a range of pressures. Based on the output signal, a clinician is able to adjust the respiratory support device to provide the pressurized airflow with a pressure outside of that range of pressures.

In an embodiment, the sensor input is adapted to receive sensor signal from a sensor external to the respiratory support device. The user input is adapted to receive the user signal from a device external to the respiratory support device.

According to the embodiment, the sensor is external to the respiratory support device. The sensor is a different device than the respiratory support device. For example, the sensor is a wearable device adapted to be worn by the subject, such as at the wrist, at the chest, or at the neck. For example, the sensor is adapted to be arranged near or at the bed of the subject. For example, the sensor has a wireless connection with the respiratory support device. The device that provides the user signal to the respiratory support device is a different device than the respiratory support device. For example, the device is a mobile device, or a user input device, or a wearable device. For example, the device has a wireless connection with the respiratory support device.

In an embodiment, the sensor signal is representative of a cardiac parameter and/or a respiratory parameter of the subject.

According to the embodiment, a cardiac parameter and/or a respiratory parameter is used to determine the objective sleep time. Cardiac parameters and respiratory parameters can be acquired using techniques that are less obtrusive than techniques to acquire neurological parameters, such as EEG. Cardiac parameters and respiratory parameters are suited to accurately determine when the subject is awake and when the subject is asleep. This allows an accurate determination of the objective sleep time.

In a third aspect, there is provided a computer-implemented method, comprising receiving a plurality of output signals for a plurality of subjects, each of the plurality of output signals being generated via the computer-implemented method according to the first aspect. The computer-implemented method comprises classifying each of the subjects into one of multiple priority classes based on the output signals. The computer-implemented method comprises generating a further output signal representative of a further suggested action for the subjects in at least one of the multiple priority classes.

According to the third aspect, groups of subjects are classified based on their respective output signals to priority classes. By classifying the subjects to the priority classes, the clinician is able to identify the group of subjects that are most likely to stop adhering to the SDB therapy. This prompts the clinician to intervene and to provide specific action to the subjects in that priority class. For example, the specific action is to provide insomnia therapy for the group of subjects.

In a fourth aspect, there is provided a computer program product comprising instructions which, when executed by a processor, cause the method of the first aspect or the third aspect to be carried out.

### BRIEF DESCRIPTION OF THE DRAWINGS

Exemplary embodiments will now be described, by way of example only, with reference to the following Figures, in which:
Fig. 1 depicts a first embodiment according to the invention;
Fig. 2 depicts a computer-implemented method according to the first embodiment;
Fig. 3 depicts a further view of the computer-implemented method of first embodiment;
Fig. 4 depicts measures of a tendency and a variation according to a second embodiment;
Fig. 5 depicts subject parameters for the multiple sleep perception classes;
Fig. 6 depicts a third embodiment according to the invention;
Fig. 7 depicts the processor according to a fourth embodiment of the invention;
Fig. 8 depicts a further computer-implemented method according to a fifth embodiment of the invention.

### DETAILED DESCRIPTION OF EMBODIMENTS

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

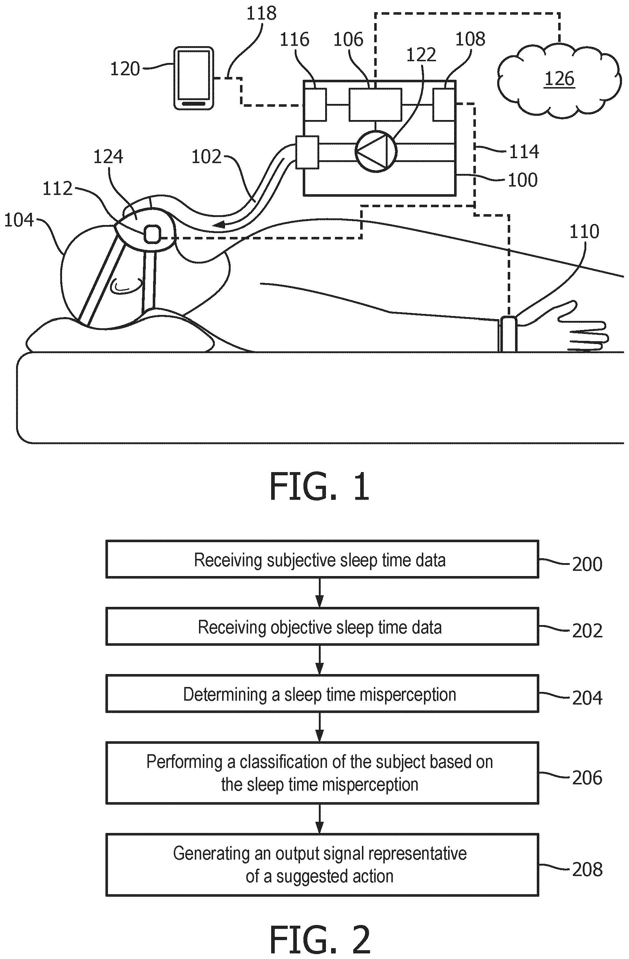

Fig. 1 depicts a first embodiment according to the invention. In the first embodiment, there is provided a respiratory support device 100 adapted to provide a pressurized airflow 102 to a subject 104. The respiratory support device 100 comprises a processor 106, a sensor input 108 and a user input 116. The processor 106 is configured to cause a computer-implemented method to be carried out. The computer-implemented method is further described with reference to Fig. 2. The sensor input 108 is adapted to receive from a sensor a sensor signal 114 representative of a sleep state of the subject 104. For example, the sensor is a PPG-sensor 110 arranged at the wrist of the subject 104. For example, the sensor is an airflow sensor 112 adapted to measure inhalation and/or exhalation by the subject 104. For example, the airflow sensor 112 is arranged in a patient interface 124 that couples to the subject 104 to provide the pressurized airflow 102 to the subject 104. The user input 116 is adapted to receive a user signal 118 representative of subjective sleep time. For example, the subject 104 provides information about the subjective sleep time to a mobile device 120. The mobile device 120 is in communication with the respiratory support device 100 and provides the user signal 118 to the user input 116. The processor 106 is connected to the sensor input 108 and the user input 116. The processor 106 is configured to determine objective sleep time data based on the sensor signal 114. The processor 106 is configured to determine the subjective sleep time data based on the user signal 118.

The processor 106 is configured to cause the computer-implemented method to be carried out. For example, the processor 106 carries out all steps of the computer-implemented method. In another example, the processor 106 causes at least one external processor to carry out at least part of the computer-implemented method. For example, the external processor is part of the cloud 126

The computer-implemented method is depicted in Fig. 2. The computer-implemented method comprises, at 200, receiving subjective sleep time data representative of the subjective sleep time of the subject 104 over multiple days. The computer-implemented method comprises, at 202, receiving objective sleep time data representative of the objective sleep time of the subject 104 over the multiple days. The computer-implemented method comprises, at 204, determining a sleep time misperception based on the subjective sleep time data and the objective sleep time data. The computer-implemented method comprises, at 206, performing a classification of the subject 104 to one of multiple sleep perception classes based on the sleep time misperception. The computer-implemented method comprises, at 208, generating an output signal representative of a suggested action based on the classification. The suggested action relates to providing sleep-related therapy to the subject 104.

For example, the computer-implemented method comprises determining sleep stages for the subject 104. The objective sleep time is based on the sleep stages. For example, the sleep stages are classified into two sleep stage classes, i.e., a wake sleep stage class and a non-wake sleep stage class. During the wake sleep stage, the subject 104 is awake. During the non-wake sleep stage, the subject 104 is asleep. For example, the sleep stages are classified into three classes. The three classes are a wake sleep stage class, a REM sleep stage class, and a non-REM sleep stage class. During the REM sleep stage class, the subject 104 is asleep and has Rapid Eye Movements, whereas during the non-REM sleep stage class, the subject 104 is asleep without Rapid Eye Movements. For example, the sleep stages are classified into four classes. The four classes are a wake sleep stage class, a REM sleep stage class, a light sleep stage class, and a deep sleep stage class. For example, the sleep stages are classified into the classes N1, N2, N3, REM and Wake.

For example, the sleep stage information is generated based on a surrogate measure of sleep. For example, the surrogate measure of sleep is based on changes in autonomic nervous system activity of the subject 104. A change in the autonomic nervous system activity is, for example, detected based on cardiac signals obtained with an appropriate sensor such as a reflective photoplethysmography (PPG) sensor, a transmissive PPG sensor or a remote PPG sensor, a ballistocardiographic sensor, or a seismocardiographic sensor. The reflective PPG sensor is, for example, arranged on the wrist or the face of the subject 104. The transmissive PPG sensor is, for example, arranged on the finger of the subject 104. The remote PPG sensor comprises, for example, an infrared camera. The ballistocardiographic sensor comprises, for example, an accelerometer or gyroscope attached to the body of the subject, or for example a pressure sensor mounted in the mattress or bed of the subject 104. The seismocardiographic sensor comprises, for example, an accelerometer mounted on the chest of the subject 104. The signals obtained by one or more of these sensors are, for example, used as input to a machine learning model trained to infer sleep stages. The input is, for example, based on manually crafted features correlating with sleep stages, such as a feature describing heart rate variability, or a feature of a time series describing heart rate progression during sleep (e.g. instantaneous heart rate). The input is, for example, input as raw data to the machine learning model.

In addition or alternatively to determining the sleep stage information as mentioned above, the sleep stage information is, for example, based on respiratory activity. Respiratory activity of the subject 104 is indicative of changes in autonomic nervous system activity associated with different sleep stages. Respiratory activity is, for example, measured with the airflow sensor 112, or a sensor adapted to measure chest movements. For example, the airflow sensor 112 comprises an oral cannula, a nasal cannula and/or a thermistor. For example, a sensor adapted to measure chest movements comprises a respiratory inductance plethysmography belt to be worn around the thorax of the abdomen. For example, the sensor adapted to measure respiratory activity comprises a pressure sensor mounted on the bed or mattress. For example, the sensor adapted to measure respiratory activity comprises a Doppler radar positioned near the subject 104. For example, the sensor for measuring respiratory activity comprises an accelerometer or a gyroscope mounted on the thorax, the abdomen and/or sternum of the subject 104.

As depicted in Fig. 1, the respiratory support device 100 comprises a pressure source 122 adapted to generate the pressurized airflow 102. In an embodiment, the processor 106 is configured to control the pressure source 122 based on the output signal.

The sensor input 108 is adapted to receive sensor signal 114 from a sensor external to the respiratory support device 100. The PPG sensor 110 is external to the respiratory support device 100. The user input 116 is adapted to receive the user signal 118 from a device external to the respiratory support device 100. The mobile device 120 is external to the respiratory support device 100.

The PPG sensor generates a sensor signal 114 that is representative of a cardiac parameter of the subject 104. The cardiac parameter is, for example, a heart rate, or heart rate variability. The airflow sensor 112 is representative of a respiratory parameter of the subject 104. The respiratory parameter is, for example, a measure of inhalation or exhalation or ventilation.

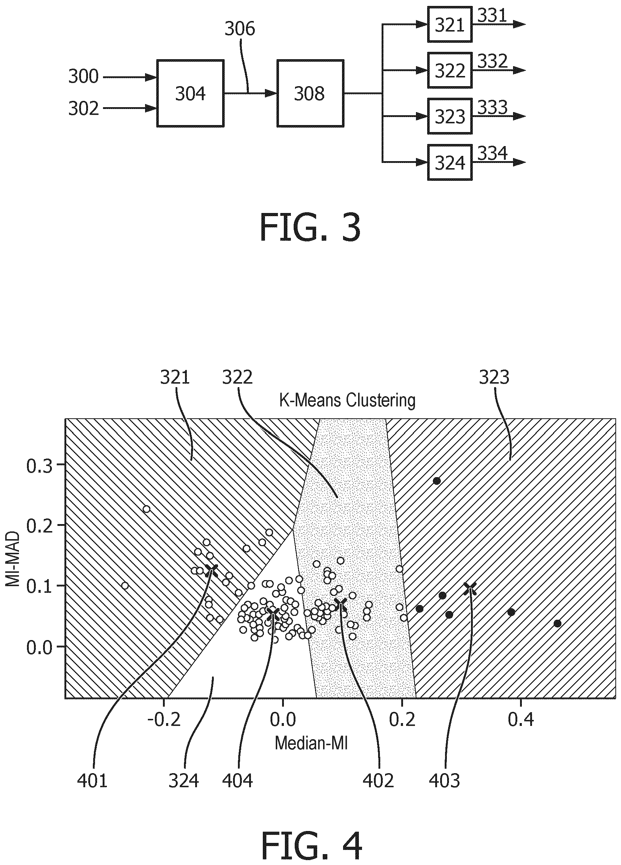

Fig. 3 depicts a further view of the computer-implemented method of the first embodiment. The processor 106 is configured to perform the computer-implemented method. The processor 106 receives the subjective sleep time data representative of the subjective sleep time of a subject 104 over multiple days. The processor 106 receives the objective sleep time data 300 representative of the objective sleep time of the subject 104 over the multiple days. At 304, the processor 106 determines a sleep time misperception based on the subjective sleep time data 302 and the objective sleep time data 300. At 308, the processor 106 performs a classification of the subject 104 to one of multiple sleep perception classes based on the sleep time misperception. Fig. 3 depicts four sleep perception classes 321-324, each relating to a different sleep time misperception. The processor 106 generates an output signal representative of a suggested action based on the classification. Fig. 3 depicts four output signals 331-334, one for each sleep perception class 321-324. Depending on the sleep perception classes 321-324 to which the subject 104 is classified, the processor 106 generates only one of the output signals 331-334 for the subject 104.

The suggested action relates to providing sleep-related therapy to the subject 104. In an embodiment, the sleep-related therapy comprises sleep disordered breathing therapy, or insomnia therapy, or a combination of sleep disordered breathing therapy and insomnia therapy.

In an embodiment, the multiple sleep perception classes comprise at least one overestimation class and at least one underestimation class. The overestimation class represents subjects indicating more subjective sleep time than the objective sleep time. The underestimation class represents subjects indicating less subjective sleep time than the objective sleep time. For example, sleep perception class 321 is the overestimation class. For example, sleep perception class 322 is the underestimation class.

In case the classification of the subject 104 is to the overestimation class 321, output signal 331 represents the suggested action relating to increasing an intensity of sleep disordered breathing therapy. For example, the sleep disordered breathing therapy comprises providing the pressurized airflow 102 to the subject 104. In case the classification is to the overestimation class 321, the output signal 331 represents the suggested action relating to providing the pressurized airflow 102 to the subject 104 at a higher pressure than in case the classification is to the underestimation class 322.

In case the classification is to the underestimation class 322, output signal 332 represents the suggested action relating to providing cognitive behavior therapy for insomnia (CBT-I). For example, in case the classification is to the underestimation class 322, output signal 332 represents the suggested the action relating to providing both cognitive behavior therapy for insomnia (CBT-I) and sleep disordered breathing therapy.

In an embodiment, the multiple sleep perception classes comprise at least one severe underestimation class. For example, the sleep perception class 323 is the severe underestimation class. The severe underestimation class 323 represents subjects indicating less subjective sleep time relative to the objective sleep time than the subjects in the underestimation class 322. Output signal 333 represents the suggested action relating to providing insomnia therapy and relates to postponing providing sleep disordered breathing therapy.

In an embodiment, the multiple sleep perception classes comprise at least one correct estimation class. For example, the sleep perception class 324 is the correct estimation class. The correct estimation class represents subjects indicating subjective sleep time matching with the objective sleep time. In case the classification is to the correct estimation class 324, the output signal 334 represents the suggested action relating to providing sleep disordered breathing therapy and relates to not providing CBT-I.

Fig. 4 depicts measures of a tendency and a variation according to a second embodiment. The second embodiment has, for example, the same elements as the first embodiment, except for the following.

In the second embodiment, the processor 106 performs the classification. The classification comprises calculating a measure of a tendency and/or a measure of a variation of the sleep time misperception over the multiple days. The measure of tendency is the median of the sleep misperception index MI according to equation (1). The measure of variation is the mean absolute deviation (MAD) of the sleep misperception index MI according to equation (1).

Each dot in the graph in Fig. 4 represents a subject 104 for whom the median and the MAD of the sleep misperception is determined based on multiple nights of measuring the objective sleep time and recording the subjective sleep time. The graph in Fig. 4 has the median on the x-axis, and the MAD on the y-axis. When plotting the values for each subject in the graph, four different groups are distinguished.

The four groups are determined using k-means clustering. K-means clustering is a known method of vector quantization that aims to partition n observations into k clusters in which each observation belongs to the cluster with the nearest centroid.

K-means clustering is applied to the data of the subjects represented by the dots in Fig. 4, and to determine four centroids 401-404. Each centroid 401-404 belongs to a cluster. The clusters represent the sleep perception classes 321-324. Each dot is Fig. 4 is classified to one of the sleep perception classes 321-324 by calculating to which centroid 401-404 the subject is closest within the feature space in terms of Euclidean distance, i.e., which centroid 401-404 is closest to a dot in terms of both the median and the MAD. The result is that each of the subjects is classified to one of the sleep perception classes 321-324.

When the processor 106 is performing the computer-implemented method, the processor 106 classifies the subject 104 to the sleep perception class 321-324 having the centroid 401-404 closest to the determined median and MAD of the subject 104. For example, the processor 106 receives information about the four centroids 401-404 and classifies the subject 104 based on the information about the four centroids 401-404.

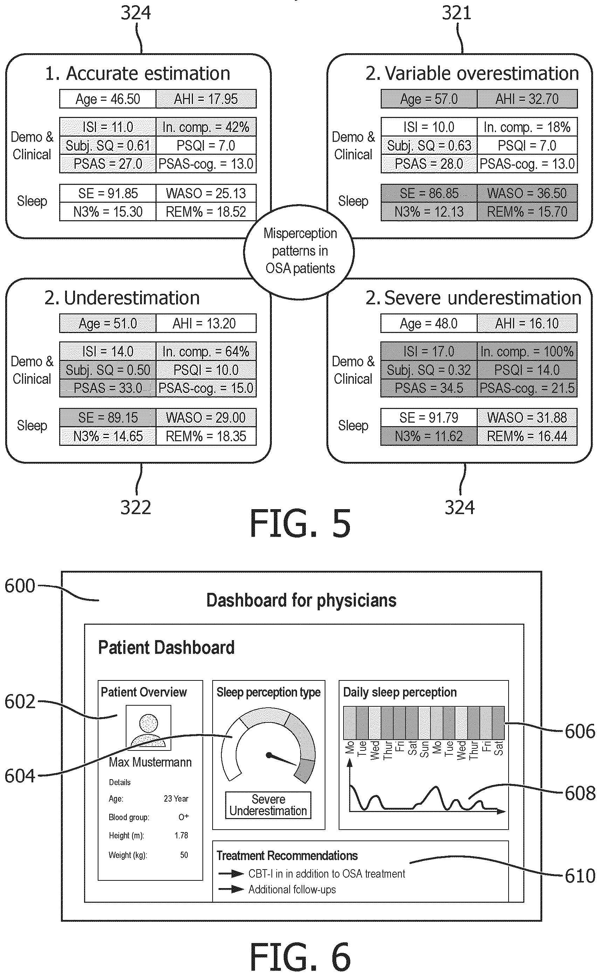

Fig. 5 depicts subject 104 parameters for the multiple sleep perception classes. The subject 104 parameters show that classifying the subject 104 to one of the sleep perception classes not only groups subjects based on sleep misperception, but also on other sleep related parameters.

The inventors have discovered that the subjects in the overestimation class 321 are older on average than the subjects in the other classes. Also, the subjects in the overestimation class 321, on average, have more severe SDB than the other classes. The severity of the SDB is represented by the AHI. The average AHI in the overestimation class 321 is 32, which is about twice as much as in the other classes. The Insomnia Severity Index, ISI, score is substantially higher in the underestimation class 322 and the severe underestimation class 323. The insomnia complaints, In. comp, is the highest in the severe underestimation class 323, and is the lowest in the overestimation class 321. The subjective sleep quality, subj. SQ, is substantially lower in the underestimation class 322 and the severe underestimation class 323 than in the other classes. The Pittsburgh Sleep Quality Index, PSQI, is substantially higher in the underestimation class 322 and the severe underestimation class 323 than in the other classes. The higher the PSQI, the more a subject 104 experiences sleep disturbance. The pre-sleep arousal scale, PSAS, for somatic arousals and cognitive arousals are substantially higher in the underestimation class 322 and the severe underestimation class 323 than in the other classes. The higher the PSAS value, the more sleep is disturbed. It is remarkable that the average sleep efficiency, i.e., the percentage of objective sleep time relative to the time in bed, is higher for the underestimation classes 322, 323 than the overestimation class 321. So the subjects in the overestimation class 321 are more awake during the time in bed than the subjects in the underestimation classes 322, 323, but do not perceive this. Also, the subjects in the overestimation class 321 suffer, on average, more from waking after sleep onset, WASO, and have less REM sleep than the subjects in the other classes. Subjects in both the overestimation class 321 and in the severe underestimation class 323 suffer from a reduced amount of deep sleep, N3-sleep, compared to the other sleep perception classes. The amounts of deep sleep and REM sleep is indicated as a percentage of the total sleep time.

Based on the information of Fig. 5, the inventors have discovered that classifying subjects based on sleep perception helps grouping subjects with common sleep parameters. By grouping the subjects in this way, the clinician is able to provide specific treatment to the subjects in a sleep perception class.

Fig. 6 depicts a third embodiment according to the invention. In the third embodiment, the output signal causes a display to generate the patient dashboard 600. The patient dashboard shows some patient overview data 602. For example, the patient overview data 602 displays the name and date of birth of the subject 104. The patient dashboard shows the sleep perception type 604. The sleep perception type 604 is based on the sleep perception class to which the subject 104 is classified. In this case, the subject 104 is classified into the severe underestimation class 323. A gauge points to the used sleep perception class to clearly indicate the sleep perception type to the clinician. The daily sleep perception is shown at 606. The daily sleep perception is based on the sleep perception only of a single night. As a result, the daily sleep perception may vary over multiple days. This variation provides insights to the clinician about the sleep perception of the subject 104. At 608, numerical values of the daily sleep perception are shown as a graph to provide insights to the clinician about the sleep misperception of the subject 104. At 610, the clinician is provided with the suggested action based on the classification of the subject 104 to one of the sleep perception classes.

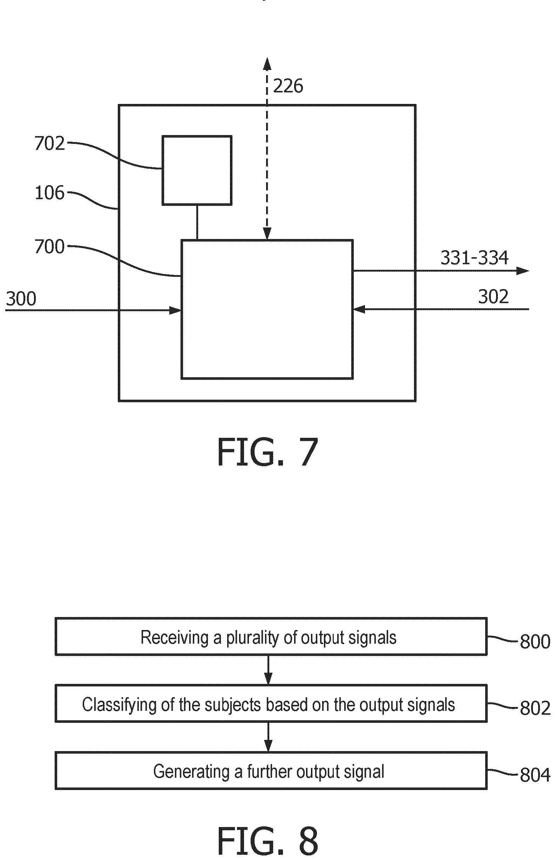

Fig. 7 depicts the processor 106 according to a fourth embodiment of the invention. The fourth embodiment has, for example, the same features as the first, second, or third embodiments, except for the following.

The processor 106 comprises a processor unit 700 and a memory 702 connected to the processing unit 700. The memory 702 comprises a computer program product comprising instructions which, when executed by the processor 106, cause the computer-implemented method of any one of the previous embodiments to be carried out. The processor unit 700 is configured to receive the subjective sleep time data 302, and the objective sleep time data 300. The processor unit 700 is configured to communicate with a processor in the cloud 126 to perform at least parts of the computer-implemented method.

Fig. 8 depicts a further computer-implemented method according to a fifth embodiment. The further computer-implemented method comprises, at 800, receiving a plurality of output signals for a plurality of subjects, each of the plurality of output signals being generated via the computer-implemented method according to the previous embodiments. The further computer-implemented method comprises, at 802, classifying each of the subjects into one of multiple priority classes based on the output signals. The further computer-implemented method comprises, at 804, generating a further output signal representative of a further suggested action for the subjects in at least one of the multiple priority classes.

For example, the processor configured to cause the further computer-implemented method to be executed comprises a processor unit and a memory connected to the processing unit. The memory comprises a computer program product comprising instructions which, when executed by the processor, cause the further computer-implemented method to be carried out.

It will be understood that the disclosed methods are computer-implemented methods. As such, there is also proposed a concept of a computer program comprising code means for implementing any described method when said program is run on the processor 106.

The skilled person would be readily capable of developing the processor 106 for carrying out any herein described method. Thus, each step of the computer-implemented method may represent a different action performed by the processor 106, and may be performed by a respective module of the processor 106.

As discussed above, the processor 106 performs data processing. The processor 106 can be implemented in numerous ways, with software and/or hardware, to perform the various functions required. The processor 106 typically employs one or more microprocessors that may be programmed using software (e.g. microcode) to perform the required functions. The processor 106 may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs). Thus, the processing system may be embodied as a digital and/or analog processing system.

In various implementations, the processing system may be associated with one or more storage media such as volatile and non-volatile computer memory such as RAM, PROM, EPROM, and EEPROM. The storage media may be encoded with one or more programs that, when executed on one or more processing systems and/or controllers, perform the required functions. Various storage media may be fixed within a processing system or controller may be transportable, such that the one or more programs stored thereon can be loaded into a processing system.

Functions implemented by the processor 106 may be implemented by a single processor or by multiple separate processing units which may together be considered to constitute a "processor". Such processing units may in some cases be remote from each other and communicate with each other in a wired or wireless manner.

A computer program may be stored/distributed on a suitable medium, such as an optical storage medium or a solid-state medium supplied together with or as part of other hardware, but may also be distributed in other forms, such as via the Internet or other wired or wireless telecommunication systems.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A computer-implemented method comprising:
receiving subjective sleep time data (302) representative of a subjective sleep time of a subject (104) over multiple days;
receiving objective sleep time data (300) representative of an objective sleep time of the subject (104) over the multiple days;
determining a sleep time misperception based on the subjective sleep time data (302) and the objective sleep time data (300);
performing a classification of the subject (104) to one of multiple sleep perception classes (321-324) based on the sleep time misperception;
generating an output signal (331-334) representative of a suggested action based on the classification,
wherein the suggested action relates to providing sleep-related therapy to the subject (104).

2. The computer-implemented method according to claim 1,
wherein the sleep-related therapy comprises sleep disordered breathing therapy, or insomnia therapy, or a combination of sleep disordered breathing therapy and insomnia therapy.

3. The computer-implemented method according to any one of the preceding claims,
wherein the multiple sleep perception classes (321-324) comprise at least one overestimation class (321) and at least one underestimation class (322),
wherein the overestimation class (321) represents subjects indicating more subjective sleep time than the objective sleep time,
wherein the underestimation class (322) represents subjects indicating less subjective sleep time than the objective sleep time.

4. The computer-implemented method according to claim 3,
wherein, in case the classification is to the overestimation class (321), the suggested action relates to increasing an intensity of sleep disordered breathing therapy,
wherein, in case the classification is to the underestimation class (322), the suggested action relates to providing cognitive behavior therapy for insomnia (CBT-I).

5. The computer-implemented method according to claim 3 or 4,
wherein the multiple sleep perception classes comprise at least one severe underestimation class (323),
wherein the severe underestimation class (323) represents subjects indicating less subjective sleep time relative to the objective sleep time than the subjects in the underestimation class (322),
wherein the suggested action relates to providing insomnia therapy and relates to postponing providing sleep disordered breathing therapy.

6. The computer-implemented method according to any one of claims 3-5, wherein, in case the classification is to the underestimation class (322), the action relates to providing both cognitive behavior therapy for insomnia (CBT-I) and sleep disordered breathing therapy.

7. The computer-implemented method according to any one of claims 3-6, wherein the sleep disordered breathing therapy comprises providing a pressurized airflow (102) to the subject,
wherein, in case the classification is to the overestimation class (321), the suggested action relates to providing the pressurized airflow (102) to the subject (104) at a higher pressure than in case the classification is to the underestimation class (322).

8. The computer-implemented method according to any one of claims 3-7,
wherein the multiple sleep perception classes comprise at least one correct estimation class (324),
wherein the correct estimation class (324) represents subjects indicating subjective sleep time matching with the objective sleep time,
wherein, in case the classification is to the correct estimation class (324), the suggested action relates to providing sleep disordered breathing therapy and relates to providing no insomnia therapy.

9. The computer-implemented method according to any one of the preceding claims, wherein performing the classification comprises:
calculating a measure of a tendency and a measure of a variation of the sleep time misperception over the multiple days;
providing a centroid of each of the multiple sleep perception classes;
classifying the subject (104) to the sleep perception class having the centroid closest to the measure of the tendency and the measure of the variation.

10. A respiratory support device (100) adapted to provide a pressurized airflow (102) to a subject, comprising:
a processor (106) configured to cause the computer-implemented method according to any one of the preceding claims to be carried out,
a sensor input (108) adapted to receive from a sensor a sensor signal (114) representative of a sleep state of the subject;
a user input (116) adapted to receive a user signal (118) representative of the subjective sleep time,
wherein the processor (106) is connected to the sensor input (108) and the user input (116),
wherein the processor (106) is configured to:
determine the objective sleep time data (300) based on the sensor signal (114); and
determine the subjective sleep time data (302) based on the user signal (118).

11. The respiratory support device (100) according to claim 10, comprising a pressure source (122) adapted to generate the pressurized airflow (102),
wherein the processor (106) is configured to control the pressure source (122) based on the output signal.

12. The respiratory support device (100) according to claim 10 or 11,
wherein the sensor input (108) is adapted to receive sensor signal (114) from a sensor (110) external to the respiratory support device (100), and
wherein the user input (116) is adapted to receive the user signal (118) from a device (120) external to the respiratory support device (100).

13. The respiratory support device (100) according to any one of claims 10-12, wherein the sensor signal (114) is representative of a cardiac parameter and/or a respiratory parameter of the subject (104).

14. A computer-implemented method, comprising:
receiving a plurality of output signals for a plurality of subjects, each of the plurality of output signals being generated via the computer-implemented method according to any one of claims 1-9,
classifying each of the subjects into one of multiple priority classes based on the output signals;
generating a further output signal representative of a further suggested action for the subjects in at least one of the multiple priority classes.

15. A computer program product comprising instructions which, when executed by a processor (106), cause the method of any one of claims 1-9 or claim 14 to be carried out.
